(19) Europäisches Patentamt
European Patent Office
Office européen des brevets

(11) Numéro de publication : **0 099 765**
**B1**

(12) # FASCICULE DE BREVET EUROPÉEN

(45) Date de publication du fascicule du brevet : **30.10.85**

(21) Numéro de dépôt : **83401040.7**

(22) Date de dépôt : **25.05.83**

(51) Int. Cl.[4] : **B 01 D 15/08**, B 01 D 53/02, C 10 G 25/00, C 07 C 7/12, C 07 C 15/24

(54) **Procédé de fractionnement de mélanges par chromatographie d'élution avec fluide en état supercritique et installation pour sa mise en œuvre.**

(30) Priorité : **03.06.82 FR 8209649**

(43) Date de publication de la demande : **01.02.84 Bulletin 84/05**

(45) Mention de la délivrance du brevet : **30.10.85 Bulletin 85/44**

(84) Etats contractants désignés : **AT BE CH DE GB IT LI LU NL SE**

(56) Documents cités :
**DE-A- 1 673 089**
**DE-A- 2 729 462**
**GB-A- 1 350 580**
**ANGEWANDTE CHEMIE, vol. 19, no. 8, août 1980, pages 575-587, Verlag Chemie GmbH., Weinheim, DE. U. VAN WASEN et al.: "Physicochemical principles and applications of supercritical fluid chromatography (SFC)"**
**ANALYTICAL CHEMISTRY, vol. 44, no. 4, avril 1972, pages 681-686, Columbus, Ohio, USA R.E. JENTOFT et al.: "Apparatus for supercritical fluid chromatography with carbon dioxide as the mobile phase"**

(73) Titulaire : **SOCIETE NATIONALE ELF AQUITAINE**
**Tour Aquitaine**
**F-92400 Courbevoie (FR)**

(72) Inventeur : **Perrut, Michel**
**35 rue du 4ème BCT**
**F-54210 St. Nicolas de Port (FR)**

(74) Mandataire : **Hirsch, Marc-Roger**
**34 rue de Bassano**
**F-75008 Paris (FR)**

**EP 0 099 765 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention se rapporte à un procédé industriel de fractionnement de mélanges de préférence liquides, mais aussi solides ou gazeux par chromatographie d'élution, l'éluant utilisé étant un fluide en état supercritique.

On sait qu'un fluide en état supercritique, c'est-à-dire dans un état caractérisé soit par une pression et une température supérieure à la pression et à la température critique pour un corps pur, soit par un point représentatif (pression, température) situé au-delà de l'enveloppe des points critiques représenté sur un diagramme (pression, température) pour un mélange, présente, pour de très nombreuses substances, un pouvoir solvant très nettement supérieur à celui observé lorsque ce fluide est à l'état gazeux, même comprimé très fortement.

Ces variations très importantes du pouvoir solvant sont d'ailleurs utilisées dans de nombreux procédés d'extraction et de fractionnement qui, dans leur mise en œuvre, s'apparentent à l'extraction liquide-liquide ou à l'extraction liquide-solide. L'un des avantages de ces procédés est notamment de permettre une séparation très aisée entre le solvant (fluide en état supercritique) et l'extrait, par simple détente.

On a par ailleurs déjà utilisé ce pouvoir solvant élevé pour mettre en œuvre des méthodes analytiques fondées sur la chromatographie d'élution, l'éluant étant un fluide en état supercritique (voir par exemple : DE-A-2 279 462 ; U. Van. Wasen et al. ANGEWANDTE CHEMIE, vol. 19, n° 8, août 1980, pages 575-587 ; R.E. Jentoft et al. ANALYTICAL CHEMISTRY, vol. 44, n° 4, avril 1972, pages 681-686). De cette manière, on a pu fractionner à des fins d'analyse des substances de masse moléculaire élevée, qui n'auraient pu être analysées par chromatographie en phase gazeuse, et cela avec du temps d'analyse beaucoup plus courts que ceux nécessaires à leur analyse par chromatographie en phase liquide.

Un objet de la présente invention est de permettre, à des fins de production industrielle, le fractionnement de mélanges en leurs différentes fractions, en utilisant la chromatographie d'élution avec pour éluant un fluide en état supercritique.

Le procédé selon l'invention pour le fractionnement d'un mélange par chromatographie d'élution, l'éluant étant un fluide en état supercritique circulant en continu à débit constant à travers la colonne de chromatographie est caractérisé en ce que l'on injecte de manière périodique, en tête de la colonne, le mélange à fractionner, les fractions du mélange étant recueillies, détectées à la sortie de la colonne et dirigées sélectivement chacune vers un séparateur d'une batterie de séparateurs dans laquelle, par réchauffage et détente, l'éluant est séparé des constituants du mélange, lesdits constituants étant recueillis en pied des séparateurs et l'éluant en tête de ceux-ci, l'éluant recueilli étant purifié, remis en état supercritique et recyclé en tête de la colonne.

Le procédé est caractérisé en outre en ce que l'introduction des fractions du mélange dans les séparateurs est effectuée de manière cyclique, selon la périodicité de l'injection du mélange, celle-ci ayant lieu sous forme de créneaux périodiques.

La présente invention a également pour objet une installation pour la mise en œuvre du procédé précité, cette installation comprenant successivement une conduite d'amenée du mélange à fractionner ; un injecteur ; une colonne de fractionnement de mélange par chromatographie d'élution ; un détecteur à la sortie duquel est raccordée une batterie de pièges, chacune des conduites d'entrée dans les pièges étant munie d'un échangeur de chaleur d'une vanne de commande de détente ; un module de recyclage d'éluant ; des moyens de recyclage de l'éluant dans l'injecteur, où il se combine au mélange à fractionner.

L'invention a en outre pour objet, une application du procédé précité à la purification de mélanges d'hydrocarbures et notamment du naphtalène brut.

Les caractéristiques et avantages de l'invention ressortiront mieux de la description suivante, donnée uniquement à titre d'exemple.

Une installation de fractionnement selon l'invention comprend, une colonne de chromatographie dont la sortie est reliée par une canalisation à l'entrée d'un détecteur, à la sortie duquel est raccordée par une canalisation une batterie de pièges disposés en parallèle, l'entrée de chacun de ces pièges étant munie d'une vanne de commande, suivie d'un échangeur de chaleur et d'une vanne de détente. La sortie de chacun des pièges est raccordée à un dispositif de purification sur lequel on reviendra ci-après.

Un conduit de recyclage raccorde en tête les pièges à un module de recyclage d'éluant dont la sortie est reliée par la canalisation à un injecteur qui comporte une entrée en mélange à fractionner et une sortie reliée par une canalisation, à travers un échangeur de chaleur, à l'entrée de la colonne.

Le module de recyclage comporte en outre une entrée pour une alimentation régulée en éluant et une sortie de purge.

Des moyens assurent l'ouverture et la fermeture cyclique des vannes en fonction de la périodicité des injections de mélange et des indications fournies par le détecteur.

Dans l'installation pour la mise en œuvre du procédé selon l'invention, l'éluant à l'état supercritique provenant du module de recyclage balaie, en continu, à débit maintenu constant, la colonne de chromatographie, après avoir reçu dans l'injecteur, des injections du mélange à fractionner, généralement sous forme liquide.

Pour une meilleure dispersion du mélange dans l'éluant, on prévoit avantageusement un mélangeur

statique au niveau de l'injecteur. On ajuste la température du fluide (éluant plus mélange) à la valeur souhaitée au moyen de l'échangeur de chaleur, puis ce fluide est introduit dans la colone, maintenue adiabatique.

De préférence, la colonne de chromatographie est constituée d'un tube cylindrique rempli d'un garnissage granulaire formé soit par un solide poreux adsorbant présentant un comportement sélectif vis-à-vis des constituants du mélange à séparer, soit par un solide poreux inerte qu'on a préalablement imprégné d'un liquide de masse moléculaire élevée présentant aussi un comportement sélectif vis-à-vis du mélange à séparer.

Selon le principe de la chromatographie d'élution, les différents constituants du mélange vont se déplacer à des vitesses différentes au sein de la colonne du fait de leur différence d'affinité avec la phase stationnaire (adsorbant ou phase imprégnée).

De la sorte, à la sortie de la colonne, on peut obtenir des « pics » séparés dans le temps de chacun des constituants, comme on peut le constater à partir des indications fournies par le détecteur. La nature de ce dernier dépend des produits utilisés : spectrophotomètre, ionisation de flamme, catharomètre, mesure de la constante diélectrique.

Ainsi, on peut réaliser l'injection du mélange à fractionner sous forme de créneaux, pendant un temps $t_i$, avec une périodicité, ou temps de cycle tc.

A la sortie de la colonne, on retrouve les pics correspondant à chacun des constituants, avec une même périodicité tc ; ces pics caractérisent les différentes fractions du mélange, $F_1$, $F_2$, ...

Ces fractions sont très simplement collectées sélectivement dans les pièges par un mouvement cyclique des vannes commandant l'entrée de ceux-ci, sous le contrôle des indications du détecteur, avec la périodicité tc.

Les pièges sont avantageusement de simples séparateurs gaz-liquide. En effet, la séparation de l'éluant et des fractions de mélange initial ainsi collectées est extrêmement simple : il suffit d'effectuer un léger réchauffage dans les échangeurs et une simple détente dans les vannes pour que le fluide en état supercritique soit porté dans un état où sa masse volumique est beaucoup plus faible que précédemment et qu'ainsi la solubilité des fractions du mélange initial soit très fortement abaissée. L'éluant sort ainsi en tête des séparateurs par la canalisation, en emportant seulement de très faibles quantités des produits, la majeure partie de ceux-ci étant évacuée en pied des séparateurs.

L'éluant sortant en tête des pièges, qui se présente à l'état gazeux, doit être reconditionné avant d'être recyclé à l'état supercritique ou liquide subcritique avec une pureté compatible à sa réutilisation comme éluant pour un nouveau cycle.

Il contient encore une faible partie des composants du mélange et on doit donc le purifier si on souhaite le recycler pour diminuer la consommation d'éluant et donc les frais opératoires. Pour cela, on pourra dans de très nombreuses applications utiliser un lit adsorbant qui captera sélectivement les impuretés contenues dans le fluide (charbon actif, tamis moléculaire, etc...) ; on utilisera classiquement plusieurs lits adsorbants dont au moins un sera utilisé à chaque instant pour purifier l'éluant pendant que les autres seront en phase de régénération selon la procédure habituellement utilisée avec ce type d'appareils. Le gaz purifié pourra alors être traité de deux façons différentes avant recyclage :

soit comprimé à l'état gazeux à la pression souhaitée pour le recyclage et refroidi dans un échangeur de chaleur à la température souhaitée pour le recyclage ;

soit condensé à l'état liquide à la pression de sortie du lit adsorbant, comprimé grâce à une pompe à la pression souhaitée puis légèrement réchauffé avant d'être introduit dans l'injecteur ou uniquement après l'injection dans l'échangeur afin d'obtenir à l'entrée de la colonne la pression et la température souhaitées.

Dans certaines applications, on pourra inclure dans le module de recyclage un lit desséchant (tamis moléculaire par exemple) si la colonne de chromatographie ou/et le mélange à séparer risque(nt) d'être altéré(s) par la présence d'eau dans l'éluant ; si l'éluant est un combustible il peut être nécessaire d'inclure un réacteur de désoxygénation catalytique afin d'éliminer toute trace d'oxygène dans l'éluant recyclé par combustion avec l'éluant. Le lit desséchant et le réacteur de désoxygénation catalytique seront de préférence disposés après le compresseur ou la pompe du module de recyclage. Si les deux sont nécessaires, le lit desséchant sera toujours placé en aval du réacteur de désoxygénation.

Dans la plupart des applications, il sera nécessaire de prévoir des réservoirs tampons de gaz issu des pièges de gaz comprimé ou de liquide subcritique après compression afin de permettre une bonne régulation du débit de l'éluant.

Afin d'éliminer l'accumulation de substances inertes présentes dans le circuit au démarrage et injectées avec le mélange, on purge en continu un faible débit tout en maintenant la quantité d'éluant présent dans le circuit par une alimentation régulée d'éluant.

A titre indicatif, la température d'entrée du mélange dans la colonne peut être avantageusement choisie entre Tc et 1,2 Tc si Tc est la température critique de l'éluant (et même dans bien des cas entre Tc et 1,05 Tc) ; la pression d'entrée du mélange doit être supérieure à la pression critique Pc de l'éluant et peut être avantageusement fixée entre 1,05 Pc et 2 Pc (et dans certains cas particuliers elle peut atteindre 3 Pc) ; la pression dans les pièges est par contre inférieure à la pression critique Pc de l'éluant et peut être choisie entre 0,5 et 0,95 Pc et avantageusement entre 0,8 et 0,9 Pc afin de limiter le coût de la recompression sans diminuer trop le rendement du piégeage.

On comprend immédiatement les avantages déterminants de ce procédé par rapport aux procédés de chromatographie préparative en phase gazeuse et en phase liquide :

la température utilisée pour le fractionnement peut être très notablement plus basse qu'en chromatographie en phase gazeuse du fait de la haute solubilité des composants du mélange dans le fluide en état supercritique et du fait qu'on n'a donc pas à vaporiser ce mélange ; on lève ainsi les obstacles majeurs liés à la fragilité des molécules et à la stabilité des phases stationnaires (surtout les phases polaires) ;

la séparation éluant-fraction est beaucoup plus aisée qu'en chromatographie en phase gazeuse (condensation sur une paroi froide avec le problème de la formation de brouillards) et qu'en chromatographie en phase liquide (distillation de mélanges très dilués par l'éluant).

D'autres avantages peuvent également être avancés, relatifs à l'économie de l'opération, liés à la faible viscosité du fluide en état supercritique et à sa grande diffusivité permettant d'utiliser des vitesses de passages élevées sans affecter l'efficacité de séparation et conduisant donc à des productivités élevées.

Dans le cas où le mélange à fractionner est initialement sous forme solide ou gazeuse, il est avantageux de le dissoudre préalablement dans un certain volume d'éluant (à l'état liquide ou supercritique selon des cas) et d'utiliser dans le procédé décrit cette solution à la façon de mélanges liquides.

Dans certaines applications, il peut être avantageux de faire varier la pression d'entrée de l'éluant dans la colonne avec une période égale à celle du cycle d'injection ; une augmentation de la pression au cours du cycle peut diminuer le temps de passage des composés les plus retenus et donc augmenter la productivité de l'installation.

On donne maintenant des exemples d'application du procédé selon l'invention :

La colonne de chromatographie qui a 1,5 m de long et 0,125 m de diamètre intérieur, est remplie d'un matériau classique (brique cuite broyée de type chromosorb P de granulométrie 200-500 µm) imprégné de polyéthylène glycol (masse molaire moyenne 6000) à un taux voisin de 20 % ; le fluide vecteur utilisé est le n-pentane (coordonnées critiques : Tc = 469,7 K et Pc = 3,33 MPa).

La température d'entrée dans la colonne est très soigneusement maintenue à 215 °C (± 0,3 °C environ) et les pressions d'entrée à 4,20 MPa (± 0,02 MPa) ; le débit de fluide est régulé avec précision à 100 kg/h. Les pièges sont maintenus à une pression de 2,2 MPa et à une température de 210 °C.

Dans ces conditions, on va mettre en œuvre la purification de naphtalène

($C_{10}H_8$ 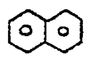);

une mesure classique du nombre de plateaux théoriques effectués par injection de naphtalène pur conduit à 450 (± 50), soit une hauteur d'étage théorique voisine de 3 mm ; cette efficacité ne peut être obtenue que grâce à un tassement très soigné de la colonne, qu'on soumet lors du remplissage à un cycle de vibrations et chocs violents, et à une régulation très soignée des paramètres opératoires.

Le naphtalène brut à purifier contient environ 5 % d'impuretés constituées d'hydrocarbures monocycliques alkylés

( 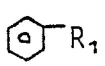, 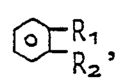, ...)

et d'hydrocarbures dicycliques alkylés

( 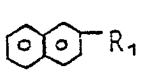, 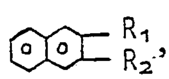, ...)

ainsi que des traces d'hydrocarbures de structures diverses, dont les points d'ébullition sont voisins de celui du naphtalène (la pureté du naphtalène brut peut être évaluée par son point de fusion soit Tf = 76,8 °C). Pour une injection durant 15 s et correspondant à 60 g pour un débit de 4 g/s, les paramètres optimaux du cycle d'injection et de piégeage ont été choisis à titre d'exemple :

— injection : 0-15 s ;
— piège produit pur : 190-220 s ;
— piège produit à recycler : 180-190 s et 220-235 s ;
— piège impuretés concentrées : 0-180 s et 235-290 s.

On obtient alors un rendement de 70 % en produit « pur » (environ 0,5 % d'impuretés, température de fusion Tf = 79,6 °C), 20 % de produit à recycler (Tf = 77,2 °C) et 10 % d'impuretés concentrées.

Compte tenu du temps de passage du composé le moins retenu (environ 150 s) et afin d'augmenter la productivité, on peut réduire le temps de cycle à 140 s en effectuant l'injection avant la sortie du « pic » précédent de la colonne, soit à t = 150 s dans le cycle précédent ; on a alors le cycle suivant :

— injection : 0-15 s ;
— piège produit pur : 40-70 s ;
— piège produit à recycler : 30-40 et 70-85 s ;
— piège impuretés : 0-30 et 85-140 s.

Les résultats obtenus pour les rendements et les puretés restent voisins de ceux décrits précédemment ; la productivité atteint avec ce cycle 1,05 kg/h de produit purifié.

Dans cette application, la purification du fluide vecteur est mise en œuvre sur un lit de charbon actif d'une contenance de 100 l environ ; on régénère par précaution tous les 5 jours environ ; le pentane et la charge contenant des quantités infimes d'eau, il n'a pas été nécessaire de mettre en œuvre un traitement de déshydratation.

Dans un deuxième exemple, on traite le même produit à purifier que précédemment.

La colonne de chromatographie qui a 0,4 m de long et 0,125 m de diamètre est remplie de silice greffée (chaînes carbonées linéaires de 18 carbones, type RP 18) d'une granulométrie moyenne de 10 μm ; le fluide vecteur utilisé est le dioxyde de carbone (coordonnées critiques : $T_c = 304{,}3$ K et $P_c = 7{,}38$ MPa).

La température d'entrée dans la colonne est soigneusement maintenue à 35 °C (± 0,1 °C) et la pression d'entrée à 15 MPa (± 0,05 MPa) ; le débit d'éluant est régulé avec précision à 115 kg/h. Les pièges sont maintenus à une pression de 6 MPa et à une température de 80 °C.

Une mesure classique du nombre de plateaux théoriques effectués dans ces conditions par injection de naphtalène pur conduit à 2 200 soit une hauteur d'étage théorique voisine de 0,16 mm ; cette efficacité ne peut être obtenue que grâce à une préparation très soignée de la colonne avec chocs, vibrations et compression du remplissage et à une régulation très soignée des paramètres opératoires.

Pour une injection de 20 s et correspondant à 50 g pour un débit de 2,5 g/s , les paramètres optimaux du cycle d'injection et de piégeage ont été choisis à titre d'exemple :

— injection : 0-20 s ;
— piège produit pur : 280-310 s ;
— piège produit à recycler : 270-280 s et 310-325 s ;
— piège impuretés concentrées : 0-270 et 325-375 s.

On obtient alors un rendement de 75 % en produit « pur » (environ 0,4 % d'impuretés, température de fusion Tf = 79,7 °C); 14 % de produit à recycler (Tf = 77 °C) et 11 % d'impuretés concentrées.

Comme dans le cas précédent, on peut augmenter la productivité de l'unité en effectuant l'injection avant la sortie du « pic » précédent de la colonne soit à t = 115 s dans le cycle précédent ; on a alors le cycle suivant :

— injection : 0-20 s,
— piège produit pur : 20-50 s,
— piège produit à recycler : 10-20 s et 50-65 s,
— piège impuretés : 0-10 s et 65-115 s,

soit une productivité égale à environ 1,15 kg/h de produit pur.

Dans cette application, la purification du fluide vecteur a été opérée dans des conditions identiques à celles de l'exemple précédent.

**Revendications**

1. Procédé de fractionnement d'un mélange par chromatographie d'élution, l'éluant étant un fluide en état supercritique circulant en continu à débit constant à travers la colonne de chromatographie caractérisé en ce que l'on injecte de manière périodique, en tête de la colonne, le mélange à fractionner, les fractions du mélange étant recueillies, détectées à la sortie de la colonne et dirigées sélectivement chacune vers un séparateur d'une batterie de séparateurs dans laquelle, par réchauffage et détente, l'éluant est séparé des constituants du mélange, lesdits constituants étant recueillis en pied des séparateurs et l'éluant en tête de ceux-ci, l'éluant recueilli étant purifié, remis en état supercritique et recyclé en tête de la colonne.

2. Procédé selon la revendication 1, caractérisé en ce que l'introduction des fractions du mélange dans les séparateurs est effectuée de manière cyclique, selon la périodicité de l'injection du mélange, celle-ci ayant lieu sous forme de créneaux périodiques.

3. Procédé selon l'une des revendications 1 et 2, caractérisé en ce qu'on purge en continu un faible débit d'éluant avant introduction dans la colonne tandis qu'on maintient constante la quantité d'éluant présent dans le circuit par alimentation régulée.

4. Procédé selon l'une quelconque des revendications 1 à 3, caractérisé en ce que la température d'entrée du mélange est choisie entre Tc et 1,2 Tc, Tc étant la température critique de l'éluant, et de préférence entre Tc et 1,05 Tc, tandis que la pression d'entrée du mélange est supérieure à la pression critique Pc de l'éluant et est comprise entre 1,05 Pc et 3 Pc, de préférence 1,05 Pc et 2 Pc.

5. Procédé selon l'une quelconque des revendications 1 à 4, caractérisé en ce que la pression dans les séparateurs est inférieure à Pc et est comprise entre 0,5 et 0,95 Pc, de préférence entre 0,8 et 0,9 Pc.

6. Procédé selon l'une quelconque des revendications 1 à 5, caractérisé en ce que l'éluant purifié est comprimé à l'état gazeux, puis refroidi afin d'être recyclé à la température et à la pression désirée.

7. Procédé selon l'une quelconque des revendications 1 à 5, caractérisé en ce que l'éluant purifié est condensé, puis comprimé dans une pompe, puis éventuellement réchauffé afin d'être recyclé à la pression et température désirée.

8. Procédé selon l'une quelconque des revendications 1 à 7, caractérisé en ce que la pression d'entrée de l'éluant dans la colonne de chromatographie est modulée en fonction du temps avec une période identique à celle du cycle d'injection.

9. Installation pour la mise en œuvre du procédé selon une quelconque des revendications 1 à 8, comportant successivement une conduite d'amenée du mélange à fractionner ; un injecteur ; une colonne de fractionnement de mélange par chromatographie d'élution ; un détecteur à la sortie duquel est raccordée une batterie de séparateurs caractérisée en ce que chacune des conduites d'entrée dans les séparateurs est munie d'un échangeur de chaleur, d'une vanne de commande et d'une vanne de détente ; et en ce que l'installation comporte en outre un module de recyclage d'éluant et des moyens de recyclage de l'éluant dans l'injecteur, où il se combine au mélange à fractionner.

10. Application du procédé selon les revendications 1 à 8, à la purification de mélanges d'hydrocarbures et notamment du naphtalène brut.

**Claims**

1. A process of fractionating a mixture by elution chromatography, the elution agent being a fluid in the supercritical state which flows continuously at a constant flow rate through the chromatography column, characterized in that it comprises periodically injecting the mixture to be fractionated into the column head, collecting and detecting at the outlet of the column the fractions of said mixture and directing said fractions each to one separator of a separator battery wherein the elution agent is separated from the constituents of the mixture by heating and expanding, collecting said constituents at the foot of said separators and collecting said eluation agent at the head thereof, purifying the collected elution agent, reconferring the supercritical state on it and recycling said elution agent into the head of said column.

2. A process according to claim 1, characterized in that the fractions of said mixture are introduced into said separators in a cyclic manner, in accordance with the periodicity of the introduction of said mixture, the latter being effected in the form of periodical crenels.

3. A process according to any one of claims 1 and 2, characterized in that a small amount of elution agent is drained continuously at a small flow rate prior to its introduction into said column, whereas the amount of elution agent present in the circuit is maintained at a constant value by controlling the feed rate.

4. A process according to any one of claims 1 to 3, characterized in that the selected inlet temperature of said mixture is comprised between Tc and 1.2 Tc, Tc being the critical temperature of the elution agent, said temperature of the mixture being preferably comprised between Tc and 1.05 Tc, while the inlet pressure of said mixture is higher than the critical pressure Pc of the elution agent, and is comprised between 1.05 Pc and 3 Pc, preferably between 1.05 Pc and 2 Pc.

5. A process according to any one of claims 1 to 4, characterized in that the pressure prevailing in the separators is lower than Pc, and is comprised between 0.5 and 0.95 Pc, preferably between 0.8 and 0.9 Pc.

6. A process according to any one of claims 1 to 5, characterized in that the purified elution agent is compressed in the gaseous state, and is then cooled with a view to being recycled at the desired pressure and temperature.

7. A process according to any one of claims 1 to 5, characterized in that the purified elution agent is condensed, and is then compressed in a pump, whereafter it is possibly heated, with a view to being recycled at the desired temperature and pressure.

8. A process according to any one of claims 1 to 7, characterized in that the inlet pressure of the elution agent introduced into the chromatography column is modulated as a function of time with a periodicity equal to that of the injection cycle.

9. An installation for carrying out the process according to any one of claims 1 to 8, comprising, in succession, a feeding conduit for the mixture to be fractionated by elution chromatography ; an injector ; an elution-chromatography mixture-fractionating column ; a detector the output of which is coupled to a battery of separators, characterized in that each one of the conduits feeding said separators is provided with a heat exchanger, a control valve and an expansion valve, and in that said installation further comprises an elution agent recycling module and means for recycling the elution agent into said injector where it is combined with the mixture to be fractionated.

10. The application of the process according to claims 1 to 8 to the purification of hydrocarbon mixtures, especially of raw naphtalene.

**Patentansprüche**

1. Verfahren zum Fraktionieren eines Gemisches durch Elutionschromatographie, wobei das Eluierungsmittel ein kontinuierlich mit konstantem Durchsatz die Chromatographiekolonne im superkritischen Zustand durchströmendes fliessfähiges Medium ist, dadurch gekennzeichnet, dass man das zu fraktionierende Gemisch periodisch in den Kolonnenkopf eingibt, wobei die Gemischfraktionen am

Kolonnenauslass aufgenommen und bestimmt und sodann je einem der eine Abscheiderbatterie bildenden Abscheider zugeleitet werden, wo das Eluierungsmittel durch Erhitzen und Entspannen von den Gemischbestandteilen getrennt wird, während diese Bestandteile am Fussteil der Abscheider und das Eluierungsmittel am Kopfteil derselben aufgenommen werden und das aufgenommene Eluierungsmittel gereinigt, sodann in den superkritischen Zustand zurückgeführt und in den Kolonnenkopf zurückgeleitet wird.

2. Verfahren nach Anspruch 1, dadurch gekennzeichnet, dass die Einleitung der Gemischfraktionen in die Abscheider zyklisch nach Massgabe der Periodizität der Einleitung des Gemisches erfolgt, welch letztere in Form periodischer Zacken durchgeführt wird.

3. Verfahren nach einem der Ansprüche 1 und 2, dadurch gekennzeichnet das man vor dem Einlassen in die Kolonne ununterbrochen eine geringe Menge von Eluierungsmittel ablässt, während man durch regulierte Zuleitung die im Kreislauf anwesende Menge von Eluierungsmittel konstant hält.

4. Verfahren nach einem der Ansprüche 1 bis 3, dadurch gekennzeichnet, dass die Einlasstemperatur des Gemisches zwischen Tc und 1,3 Tc gehalten wird, wobei Tc die kritische Temperatur des Eluierungsmittels ist, während der Einlassdruck des Gemisches höher ist, als der kritische Druck Pc des Eluierungsmittels und zwichen 1,05 Pc und 3 Pc, vorzugsweise zwischen 1,05 Pc und 2 Pc liegt.

5. Verfahren nach einem der Ansprüche 1 bis 4, dadurch gekennzeichnet, dass der in den Abscheidern herrschende Druck niedriger als Pc ist und zwischen 0,5 Pc und 0,95 Pc, vorzugsweise zwischen 0,8 Pc und 0,9 Pc liegt.

6. Verfahren nach einem der Ansprüche 1 bis 5, dadurch gekennzeichnet, dass das gereinigte Eluierungsmittel im gasförmigen Zustand verdichtet und sodann gekühlt wird, um bei dem gewünschten Druck und der gewünschten Temperatur zurückgeleitet zu werden.

7. Verfahren nach einem der Ansprüche 1 bis 5, dadurch gekennzeichnet, dass das Eluierungsmittel kondensiert und sodann in einer Pumpe verdichtet wird, um dann gegebenenfalls zwecks Rückleitung bei der gewünschten Temperatur und dem gewünschten Druck erhitzt zu werden.

8. Verfahren nach einem der Ansprüche 1 bis 7, dadurch gekennzeichnet, dass der Druck des Eluierungsmittels beim Einlassen in die Chromatographiekolonne gemäss einer Periodizität, die derjenigen des Einlasszyklus entspricht, in Abhängigkeit von der Temperatur moduliert wird.

9. Einrichtung für die Durchführung des Verfahrens nach einem der Ansprüche 1 bis 8, die folgende hintereinander angeordnete Einrichtungsteile umfasst : eine Zuleitung für das zu fraktionierende Gemisch ; ein Einlassorgan ; eine Fraktionierungssäule zum Fraktionieren eines Gemisches durch Elutionschromatographie ; einen Fühler, an dessen Ausgang eine Abscheiderbatterie angeschlossen ist, dadurch gekennzeichnet, dass jede der Zuleitungen zu den Abscheidern mit einem Wärmeaustauscher, einem Steuerventil und einem Entspannungsventil versehen ist, und dass die Einrichtung ferner ein Eluierungsmittel-Rückführungsmodul und Mittel zur Rückführung des Eluierungsmittels zum Einlassorgan besitzt, wo das Eluierungsmittel mit dem zu fraktionierenden Gemisch vereinigt wird.

10. Anwendung des Verfahrens nach den Ansprüchen 1 bis 8 auf die Reinigung von Kohlenwasserstoffgemischen, insbesondere die Reinigung von Rohnaphtalin.